# EUROPEAN PATENT APPLICATION

(11) **EP 4 361 318 A1**
(43) Date of publication of application: **01.05.2024**
(21) Application number: 22827514.5
(22) Date of filing: 20.06.2022
(51) Int. Cl.: C25B 15/08, C01B 13/02, C25B 1/04, A61M 16/12

(54) **OXYGEN-HYDROGEN INTEGRATED MACHINE**

(30) Priority: 21.06.2021 CN 202110687390
(71) Applicant: Shenzhen Sanai Health Technology Co., Ltd, Shenzhen, Guangdong 518000 (CN)
(72) Inventor: LIN, Loufei, Yueqing, Zhejiang 325600 (CN)
(74) Representative: Zaboliene, Reda
(86) International application number: PCT/CN2022/099775
(87) International publication number: WO 2022/268020

(57) **Abstract**

Disclosed in the present invention is an oxygen-hydrogen integrated machine including a machine housing (1), where a molecular sieve oxygen generation module and an electrolytic hydrogen generation module are provided in the machine housing (1), an oxygen-hydrogen interface (5) is provided on one side of the machine housing (1), the molecular sieve oxygen generation module and the electrolytic hydrogen generation module respectively output oxygen and hydrogen at the same gas pressure by means of an oxygen pressure regulating valve (28) and a hydrogen pressure regulating valve (17), an oxygen flow limiting hole (30) and a hydrogen flow limiting hole (18) are respectively provided at an output end of the oxygen pressure regulating valve (28) and an output end of the hydrogen pressure regulating valve (17), and the oxygen and the hydrogen are in communication with the oxygen-hydrogen interface (5) after passing through the oxygen flow limiting hole (30) and the hydrogen flow limiting hole (18); and a ratio of the cross-sectional areas of the oxygen flow limiting hole (30) and the hydrogen flow limiting hole (18) is greater than 24:1. By means of the flow limiting holes, a defined volume, and a defined output, the volume ratio of the oxygen to the hydrogen in each link of the device is greater than 24:1, so that a state of uncontrollable risk is avoided when the oxygen and the hydrogen are mixed.

## Description

### FIELD OF TECHNOLOGY

The present invention relates to the field of assisted breathing apparatus, and in particular to an oxygen-hydrogen integrated machine.

### BACKGROUND

"Hydrogen", i.e., "hydrogen molecules", is the smallest molecule in nature that is extremely penetrable and diffuses through the skin and mucosa into any organ, tissue, cell, mitochondria and nucleus of a human body. Hydrogen has an ideal selective antioxidant effect, may selectively and efficiently remove malignant free radicals-the source of diseases and aging, realize internal environment balance from the most basic cell and body fluid level of human body, start to stimulate self-repair mechanism of the human body, and improve health status comprehensively.

At present, there are two ways for outputting hydrogen by a device for generating hydrogen and oxygen through electrolysis for an oxygen-hydrogen machine, one way is pure hydrogen output, and the other way is oxygen-hydrogen mixed output. A hydrogen suction tube having small apertures for pure hydrogen output is inserted into the nostrils, although an amount of air will be sucked into the gaps beside, a user is prone to insufficient oxygen supply and there would be a low oxygen phenomenon; the oxygen-hydrogen mixed output can solve the problem of low oxygen, but the volume ratio of hydrogen to oxygen in the oxygen-hydrogen mixing mode is 2:1, and according to the theoretical calculation, the explosion limit of hydrogen is 4%-75.6% (volume concentration), so the existing oxygen-hydrogen mixing mode is effective, but the safety is not high. In addition, the oxygen produced by electrolysis is less and the pressure is insufficient, making it difficult to meet the user's demand for oxygen.

### SUMMARY

A technical problem to be solved by the present invention is to provide an oxygen-hydrogen integrated machine capable of absolutely and safely outputting an oxygen-hydrogen mixture so as to satisfy the user's demand for an oxygen-hydrogen mixed gas.

In order to solve the above technical problem, the technical solution of the present invention is as follows:
an oxygen-hydrogen integrated machine including a machine housing, wherein a molecular sieve oxygen generation module and an electrolytic hydrogen generation module are provided in the machine housing, an oxygen-hydrogen interface is provided on one side of the machine housing, the molecular sieve oxygen generation module and the electrolytic hydrogen generation module respectively output oxygen and hydrogen at the same gas pressure by means of an oxygen pressure regulating valve and a hydrogen pressure regulating valve, an oxygen flow limiting hole and a hydrogen flow limiting hole are respectively provided at an output end of the oxygen pressure regulating valve and an output end of the hydrogen pressure regulating valve, and the oxygen and the hydrogen are in communication with the oxygen-hydrogen interface after passing through the oxygen flow limiting hole and the hydrogen flow limiting hole; and a ratio of the cross-sectional areas of the oxygen flow limiting hole and the hydrogen flow limiting hole is greater than 24:1.

Further, the molecular sieve oxygen generation module and the electrolytic hydrogen generation module are respectively provided with an oxygen storage tank and a hydrogen storage tank, and the ratio of the volumes between the oxygen storage tank and the hydrogen storage tank is greater than 24: 1; an output end of the oxygen storage tank is provided with an oxygen pressure regulating valve, and an output end of the hydrogen storage tank is provided with the hydrogen pressure regulating valve.

Further, a ratio of an oxygen output of the molecular sieve oxygen generation module to a hydrogen output of the electrolytic hydrogen generation module is greater than 24:1.

Specifically, a gas-mixing valve is provided on the oxygen-hydrogen interface, input ends of the gas-mixing valve are respectively connected with oxygen and hydrogen, and an oxygen-hydrogen mixed gas is formed at the oxygen-hydrogen interface, and the volume concentration of hydrogen in the oxygen-hydrogen mixed gas is not greater than 4%.

Further, an oxygen safety valve is provided between the oxygen storage tank and the oxygen pressure regulating valve, and a hydrogen safety valve is provided between the hydrogen storage tank and the hydrogen pressure regulating valve.

Alternatively, an output end of the gas-mixing valve is connected with a flow regulating valve for regulating an output of the oxygen-hydrogen mixed gas.

Alternatively, the oxygen-hydrogen interface is provided with a humidifier or an atomizer. Specifically, the humidifier or atomizer is provided at the output end of the flow regulating valve.

Further, a control circuit board and a power supply device are provided in the machine housing, and the control circuit board is connected with a display screen, a power switch and a light-emitting diode (LED) indicator lamp provided on one side of the machine housing; the power supply device and the control circuit board are respectively connected with the molecular sieve oxygen generation module and the electrolytic hydrogen generation module.

Further, the molecular sieve oxygen generation module includes a compressor, a gas production filter connected with an input end of the compressor, and a plurality of molecular sieves connected with an output end of the compressor via a solenoid valve group, and the molecular sieves are connected with the oxygen storage tank.

Further, the plurality of molecular sieves are provided in the machine housing via a mounting vertical plate, the compressor is provided in a mounting frame, and the solenoid valve group, a plurality of condensing fans and a condenser are provided on the mounting frame; the control circuit board and the power supply device are provided on the mounting frame via a circuit mounting board.

Further, the electrolytic hydrogen generation module comprises an electrolytic tank, a pure water tank provided on an upper side of the electrolytic tank via a water tank mounting board, and a hydrogen water separator, wherein the pure water tank is connected with the electrolytic tank via a pure water pipe, a solid polymer electrolyte (SPE) membrane, a sintered electrolytic anode and a sintered electrolytic cathode are provided in the electrolytic tank, the SPE membrane divides the tank body into a hydrogen chamber and an oxygen chamber, and the hydrogen chamber is in communication with the hydrogen water separator.

Further, the electrolytic hydrogen generation module further comprises an oxygen-water separator, the oxygen chamber being in communication with the oxygen-water separator, the oxygen-water separator delivering oxygen to a gas production filter of the molecular sieve oxygen generation module via an oxygen pipe.

Alternatively, a water level sensor and a water temperature sensor are provided corresponding to the pure water tank, and the water level sensor and the water temperature sensor are connected with the control circuit board.

Alternatively, the electrolytic hydrogen generation module further includes an ion exchange resin filter element, a circulation loop is formed between the pure water tank and the ion exchange resin filter element, a water quality detection head is provided on the circulation loop, and the water quality detection head is connected with the control circuit board.

Alternatively, a voltage sensor for monitoring an operating voltage of a single cell and a temperature sensor for monitoring a temperature of the electrolytic tank are provided corresponding to the electrolytic tank, and the voltage sensor and the temperature sensor being connected with the control circuit board.

Alternatively, an orientation sensor for monitoring an inclination angle of the machine housing is provided in the machine housing, the orientation sensor being connected with the control circuit board.

Alternatively, an oxygen check valve is provided at an input end of the oxygen storage tank, and a hydrogen check valve is provided at an input end of the hydrogen storage tank.

Preferably, the control circuit board is provided to be connected with a wireless communication device, and the wireless communication device is one or more of a Bluetooth communication module, a wireless radio frequency (RF) communication module, and a Wi-Fi communication module. Preferably, a Bluetooth 4.0 communication module is used for realizing data communication with a mobile terminal, and can be connected with a cloud server via the mobile terminal or directly connected with the cloud server.

With the above-mentioned technical solution, the oxygen-hydrogen integrated machine of the present invention is provided with a molecular sieve oxygen generation module and an electrolytic hydrogen generation module, wherein pressure regulating valves are respectively provided at the output ends of oxygen and hydrogen so that the output pressures of oxygen and hydrogen are the same, and then oxygen and hydrogen are mixed via a flow limiting hole; a volume ratio of oxygen to hydrogen is greater than 24: 1, and the oxygen-hydrogen mixed gas is in an absolutely safe state by using the physical structure characteristics of the flow limiting hole; secondly, the volume ratio of the oxygen storage tank to the hydrogen storage tank is set to be greater than 24: 1, and the ratio of the input oxygen and hydrogen is ensured to be in an absolute safe state before the oxygen and hydrogen are mixed; thirdly, the ratio of the hydrogen output of the molecular sieve oxygen generation module and the electrolytic hydrogen generation module is greater than 24: 1, so that the ratio between the oxygen generation amount and the hydrogen production amount of the device is in an absolute safe state, and the oxygen of the electrolytic hydrogen generation module is input to a gas production end of the molecular sieve oxygen generation module, improving the oxygen generation efficiency and further increasing the safety factor of the device, and avoiding a dangerous uncontrollable state of the existing oxygen generator and hydrogen generator combination or oxygen and hydrogen generator when oxygen and hydrogen are mixed by the above multiple safety restrictions.

### BRIEF DESCRIPTION OF THE DRAWINGS

In order to explain the technical solutions in the embodiments or the prior art of the present invention more clearly, a brief description will be given below with reference to the accompanying drawings which are used in the description of the embodiments or the prior art, and it is obvious that the drawings in the description below are merely some embodiments of the present invention, and it would have been obvious for a person skilled in the art to obtain other drawings according to these drawings without involving any inventive effort.
FIG. 1 is a main view structural diagram of an oxygen-hydrogen integrated machine according to the present invention;
FIG. 2 is a side view structural diagram of an oxygen-hydrogen integrated machine according to the present invention;
FIG. 3 is a left cross-sectional structural diagram of an oxygen-hydrogen integrated machine according to the present invention;
FIG. 4 is a right cross-sectional structural diagram of an oxygen-hydrogen integrated machine according to the present invention;
FIG.5 is a first structural diagram of a molecular sieve oxygen generation module according to the present invention;
FIG. 6 is a second structural diagram of a molecular sieve oxygen generation module according to the present invention;
FIG. 7 is a first three-dimensional structural diagram of an electrolytic hydrogen generation module according to the present invention;
FIG. 8 is a second three-dimensional structural diagram of an electrolytic hydrogen generation module according to the present invention;
FIG. 9 is a diagram showing the structural principle of an oxygen-hydrogen integrated machine according to the present invention;

In the figure, 1-machine housing, 2-display screen, 3-power switch, 4-LED indicator lamp, 5-oxygen-hydrogen interface, 6-humidifier/atomizer, 7-switch board, 8-control circuit board, 81-wireless communication device, 82-orientation sensor, 83-power-off alarm device; 9-pure water tank, 91-water level/water temperature sensor, 92-ion exchange resin filter element, 93-water quality detection head; 10-water tank cover, 11-water tank mounting board, 12-electrolytic tank, 121-voltage sensor, 122-temperature sensor; 13-hydrogen water separator, 14-pure water pipe, 15-hydrogen pipe, 16-hydrogen storage tank, 161-hydrogen pressure sensor, 162-hydrogen safety valve; 17-hydrogen pressure regulating valve, 18-hydrogen flow limiting hole, 19-oxygen pipe, 20-compressor, 21-gas production filter, 22-gas production muffler, 23-mounting vertical plate, 24-molecular sieve, 25-solenoid valve group, 26-exhaust muffler assembly, 27-oxygen storage tank, 271-oxygen pressure sensor, 272-oxygen safety valve, 273-oxygen concentration sensor; 28-oxygen pressure regulating valve, 29-oxygen check valve, 30-oxygen flow limiting hole, 31-gas-mixing valve, 32-hydrogen check valve, 33-gas production filter screen, 34-mounting frame, 35-condensing fan, 36-condenser, 37-circuit mounting board, 38-power supply device, 39-electrolytic tank radiating fan, 40-oxygen-water separator, 41-flow regulating valve, 42-water return pipe.

### DESCRIPTION OF THE EMBODIMENTS

Reference will now be made in detail to the embodiments of the present invention, examples of which are illustrated in the accompanying drawings. It is to be understood that the description of these embodiments is intended to aid in the understanding of the present invention, and is not intended to limit the scope of the present invention. Further, the technical features involved in the various embodiments of the present invention described below can be combined with each other as long as they do not conflict with each other.

### Embodiment 1:

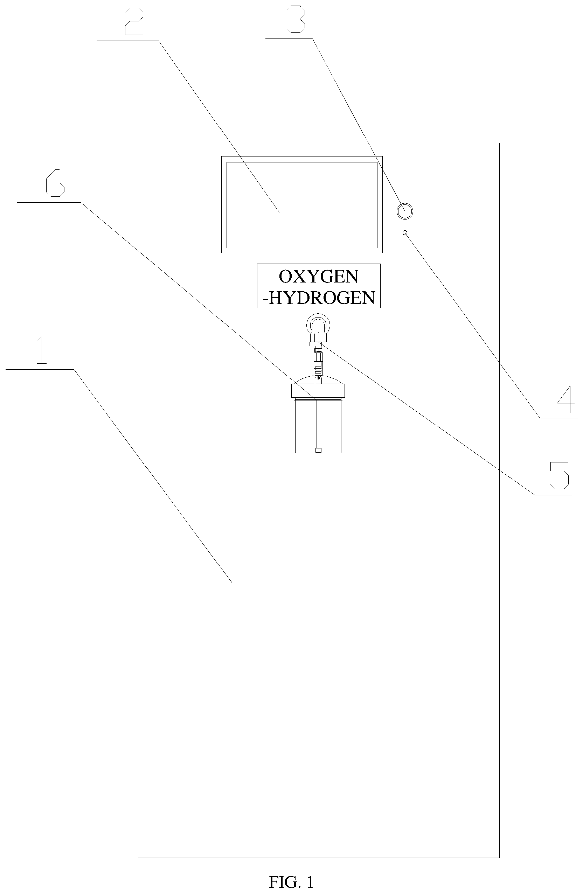

As shown in FIGS. 1-4 and 9, an embodiment of the present invention provides an oxygen-hydrogen integrated machine including a machine housing 1, wherein a molecular sieve oxygen generation module and an electrolytic hydrogen generation module are provided in the machine housing 1, an oxygen-hydrogen interface 5 is provided on one side of the machine housing 1, the molecular sieve oxygen generation module and the electrolytic hydrogen generation module respectively output oxygen and hydrogen at the same gas pressure by means of an oxygen pressure regulating valve 28 and a hydrogen pressure regulating valve 17, an oxygen flow limiting hole 30 and a hydrogen flow limiting hole 18 are respectively provided at an output end of the oxygen pressure regulating valve 28 and an output end of the hydrogen pressure regulating valve 17, and the oxygen and the hydrogen are in communication with the oxygen-hydrogen interface 5 after passing through the oxygen flow limiting hole 30 and the hydrogen flow limiting hole 18; and a ratio of the cross-sectional areas of the oxygen flow limiting hole 30 and the hydrogen flow limiting hole 18 is greater than 24:1.

Specifically, a gas-mixing valve 31 is provided on the oxygen-hydrogen interface 5, the input ends of the gas-mixing valve 31 are respectively connected with oxygen and hydrogen, and an oxygen-hydrogen mixed gas is formed at the oxygen-hydrogen interface 5, and the volume concentration of hydrogen in the oxygen-hydrogen mixed gas is not greater than 4%. The gas-mixing valve 31 may be a three-way valve, or an oxygen-hydrogen mixing tank may be used to mix oxygen and hydrogen in the tank body and then output.

When in use, since the oxygen and the hydrogen output by the molecular sieve oxygen generation module and the electrolytic hydrogen generation module respectively have the same gas pressure using the pressure regulating valve, then the amount of gas passing through the flow limiting hole in a unit time is related to a size of an aperture, and when the ratio of the cross-sectional areas between the oxygen flow limiting hole and the hydrogen flow limiting hole is greater than 24:1, that is to say, the hydrogen concentration in the oxygen-hydrogen mixed gas will not be greater than 4%; it is ensured that the oxygen-hydrogen mixed gas is in an absolute safe state, and avoids regulating the output ratio of oxygen and hydrogen by using a flow regulating valve. The flow regulating valve cannot ensure absolute stability and safety, and manual regulation easily makes errors, so that the device is in a dangerous and uncontrollable state, while the flow limiting hole is used to limit the oxygen and hydrogen ratio within a safe and controllable range through a physical structure, thus achieving an absolute safe output state of the device.

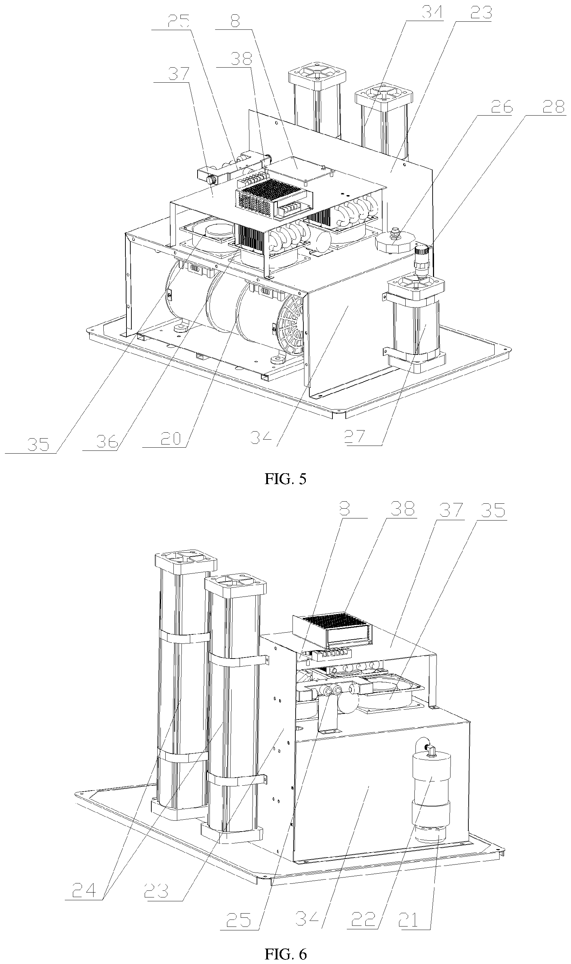

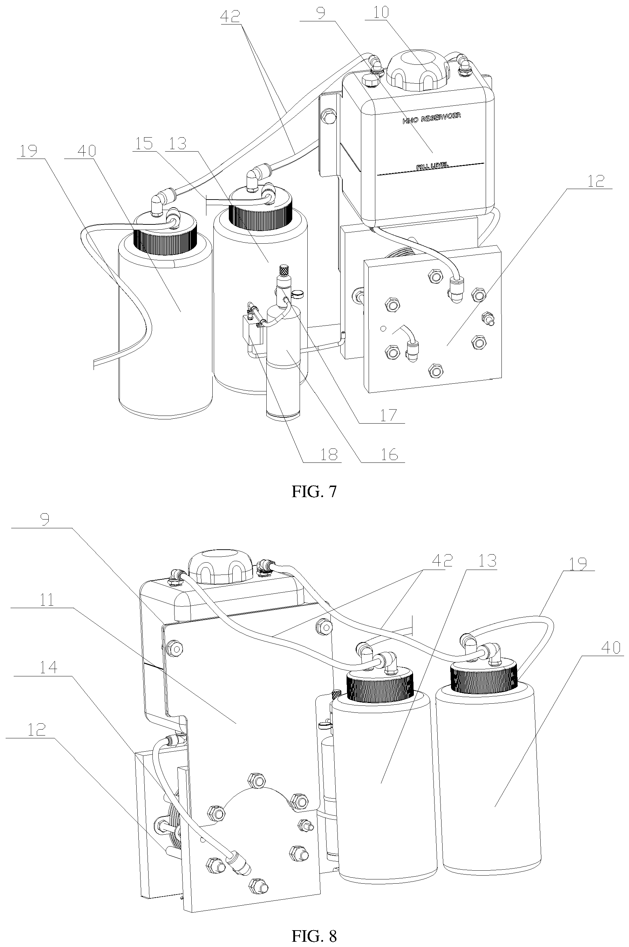

As shown in FIGS. 6 and 7, the molecular sieve oxygen generation module and the electrolytic hydrogen generation module are respectively provided with an oxygen storage tank 27 and a hydrogen storage tank 16, and the ratio of the volumes between the oxygen storage tank 27 and the hydrogen storage tank 16 is greater than 24: 1; the output end of the oxygen storage tank 27 is provided with the oxygen pressure regulating valve 28, and the output end of the hydrogen storage tank 16 is provided with the hydrogen pressure regulating valve 17. By setting the volume ratio between the oxygen storage tank 27 and the hydrogen storage tank 16 to be greater than 24: 1, that is to say, the volume concentration of oxygen and hydrogen stored in the oxygen storage tank 27 and the hydrogen storage tank 16 before mixing is not greater than 4%, it is absolutely safe that direct mixing occurs between the oxygen storage tank 27 and the hydrogen storage tank 16.

Preferably, a ratio of an oxygen output of the molecular sieve oxygen generation module to a hydrogen output of the electrolytic hydrogen generation module is greater than 24:1. Even if the oxygen output from the molecular sieve oxygen generation module is directly mixed with the hydrogen output from the electrolytic hydrogen generation module, the hydrogen concentration will be no more than 4%, and it is in an absolutely safe state.

Hydrogen has the function of carrying oxygen, oxygen is recognized as an effective treatment for respiratory diseases. As the smallest molecule in nature, hydrogen can enter the deep lung more easily than oxygen. The strong penetration can make hydrogen diffuse into the whole body and enter into tanks easily to relieve lung inflammation through the inhibition and elimination effect of hydrogen on inflammation. Oxygen therapy with hydrogen is therefore more effective than oxygen alone.

Hydrogen molecules have been proved to be very safe for human body, but there is an unavoidable safety problem for mixing hydrogen and oxygen, that is, combustion range and detonation range of the mixing of hydrogen and air are 4-75.6% (V/V) and 18-59% (V/V), respectively, the combustion range and detonation range of the mixing of hydrogen and oxygen are 4.65-94% (V/V) and 18.3-58.9% (V/V), respectively, and the ignition energy is only 0.02 mj and the flame velocity is 270 cm/s. When the aforementioned mixed gas encounters an open flame or an electrostatic spark, it burns and explodes. The hydrogen/oxygen ratio generated by the hydrogen-oxygen atomizer that has been approved by medical devices is 2:1, and the concentration of hydrogen is just within this explosion range, so it has great potential safety hazard in non-professional fields such as at home. This safety problem cannot be solved by the existing mixed gas of hydrogen and oxygen produced by electrolyzing water, because the ratio of hydrogen to oxygen produced by electrolyzing water is always 2:1, oxygen will be discharged from some devices with only hydrogen being reserved, pure hydrogen is also flammable gas, and pure hydrogen is not conducive to the treatment of respiratory diseases, and patients also have the risk of suffocation. In order to provide both oxygen and hydrogen to the patient better and more safely, the combination of the advantages of both gases must be designed to eliminate these hidden safety hazards.

The oxygen-hydrogen integrated machine of the present invention completely solves the above-mentioned safety problem and achieves intrinsic safety. In the technical solution of the present invention, a PSA pressure swing adsorption small-sized molecular sieve oxygen generation method and hydrogen production by water electrolysis are used, and the volume concentration of hydrogen in the hydrogen-oxygen mixed gas supplied to a user is ensured to be within 4% by means of physical current limiting, thus completely eliminating the risk of explosion. Even in a narrow space such as a vehicle or a tent in which oxygen and hydrogen of the oxygen-hydrogen integrated machine are directly discharged, since the hydrogen concentration is always lower than 4%, there is no risk of combustion or explosion, and in addition, when a user sleeps, the oxygen-hydrogen mixed gas is directly discharged into a quilt to be collected, and an electrostatic spark is generated, so that the oxygen-hydrogen mixed gas cannot be ignited, thereby causing a danger.

Specifically, the present invention uses the following technical solutions to achieve intrinsic safety: Example 7: oxygen-hydrogen integrated machine:
1. Energy Limit: the output of oxygen produced by PSA pressure swing adsorption small molecular sieve is ≥ 6720ml, the output of hydrogen produced by electrolysis is < 280ml, and the ratio of the output of oxygen produced by PSA pressure swing adsorption small molecular sieve to the output of hydrogen produced by electrolysis is limited from the source to be greater than 24:1.
2. The ratio of the volume of the oxygen storage tank provided at the oxygen output end of the PSA pressure swing adsorption small molecular sieve to the volume of the hydrogen storage tank provided at the hydrogen output end of the electrolytic hydrogen production is greater than 24:1.
3. An oxygen pressure regulating valve and a hydrogen pressure regulating valve are respectively installed on the oxygen storage tank and the hydrogen storage tank, so that oxygen and hydrogen have the same pressure when they are output; an oxygen flow limiting hole and a hydrogen flow limiting hole are respectively provided at the output ends of the oxygen pressure regulating valve and the hydrogen pressure regulating valve, and the ratio of the cross-sectional areas between the oxygen flow limiting hole and the hydrogen flow limiting hole is greater than 24:1;

Through the special design of the above-mentioned three methods, the volume concentration of hydrogen in the oxygen-hydrogen mixed gas output by the oxygen-hydrogen integrated machine is kept constant at not more than 4%. Even if a fire source is encountered, it will not cause an explosion.

The oxygen-hydrogen integrated machine according to an embodiment of the present invention can completely eliminate the safety risks of the oxygen-hydrogen mixed gas, so that the oxygen-hydrogen mixed treatment scheme can be more rapidly popularized. The oxygen-hydrogen integrated machine of the present embodiment has a stronger permeability and has enhanced an anti-inflammatory effect of hydrogen compared with the existing oxygen generator, has a better therapeutic effect on lung diseases, is safer and has a lower cost than the existing oxygen-hydrogen mixed atomizing machine, and can be quickly released to a region with a serious global epidemic after being mature and mass-produced, thus saving more lives.

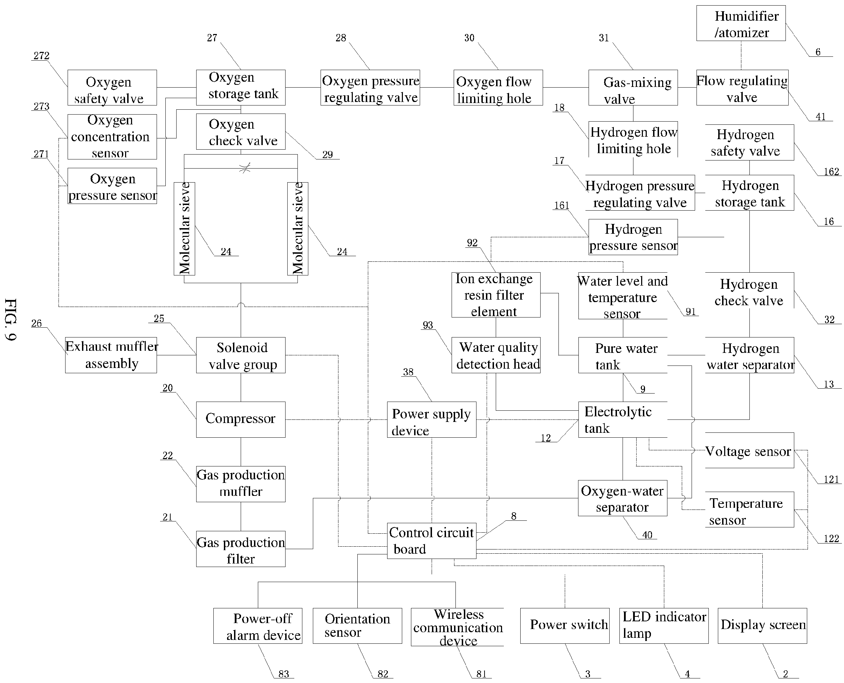

Preferably, as shown in FIG. 9, an oxygen safety valve 272 is provided between the oxygen storage tank 27 and the oxygen pressure regulating valve 28, and a hydrogen safety valve 162 is provided between the hydrogen storage tank 16 and the hydrogen pressure regulating valve 17. The oxygen safety valve 272 and the hydrogen safety valve 162 prevent the overpressure of the oxygen storage tank 27 and the hydrogen storage tank 16. Alternatively, an output end of the gas-mixing valve 31 is connected with a flow regulating valve 41 for regulating an output of the oxygen-hydrogen mixed gas.

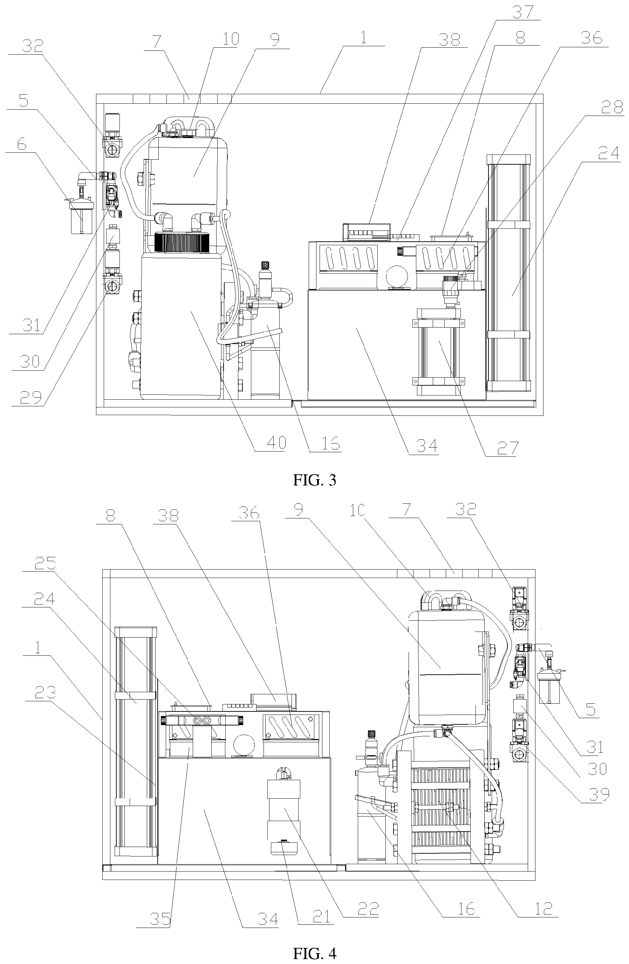

Alternatively, the oxygen-hydrogen interface 5 is provided with a humidifier or atomizer 6. Specifically, the humidifier or atomizer 6 is provided at the output end of the flow regulating valve. As shown in FIGS. 3-6, a control circuit board 8 and a power supply device 38 are provided in the machine housing 1, and the control circuit board 8 is connected with a display screen 2, a power switch 3 and an LED indicator lamp 4 provided on one side of the machine housing 1; the power supply device 38 and the control circuit board 8 are respectively connected with the molecular sieve oxygen generation module and the electrolytic hydrogen generation module.

As shown in FIGS. 3, 4, 5 and 6, the molecular sieve oxygen generation module includes a compressor 20, a gas production filter 21 connected with an input end of the compressor 20, a plurality of molecular sieves 24 connected with an output end of the compressor 20 via a solenoid valve group 25, and the molecular sieves 24 are connected with the oxygen storage tank 27. An oxygen concentration sensor 273 and an oxygen pressure sensor 271 are provided corresponding to the oxygen storage tank 27, wherein the oxygen concentration sensor 273 and the oxygen pressure sensor 271 are connected with the control circuit board 8 for monitoring the oxygen concentration and pressure parameters of the oxygen storage tank 27.

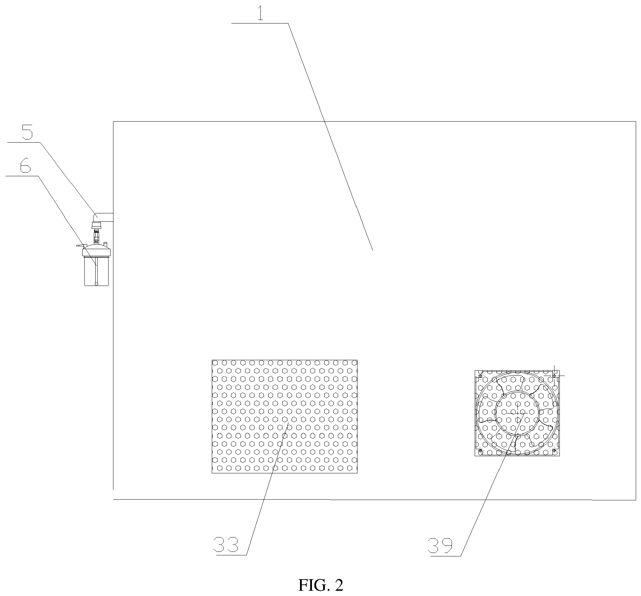

Alternatively, the gas production filter 21 is provided with a gas production muffler 22, and an exhaust muffler assembly 26 is also provided to be connected with the solenoid valve group 25. Alternatively, as shown in FIG. 2, a gas production filter screen 33 is provided on the machine housing 1 corresponding to the gas production filter 21. Specifically, the plurality of molecular sieves 24 are provided in the machine housing 1 through a mounting vertical plate 23, and the compressor 20 is provided in a mounting frame 34 on which the solenoid valve group 25, a plurality of condensing fans 35 and a condenser 36 are provided.

Specifically, the control circuit board 8 and the power supply device 38 are provided on the mounting frame 34 through a circuit mounting board 37. At least one oxygen storage tank 27 is provided at one side of the mounting frame 34, and an oxygen pressure regulating valve 28 is provided at an output end of the oxygen storage tank 27.

As shown in FIGS. 7 and 8, the electrolytic hydrogen generation module includes an electrolytic tank 12, a pure water tank 9 provided on an upper side of the electrolytic tank 12 via a water tank mounting board 11, and a hydrogen water separator 13, wherein the pure water tank 9 is connected with the electrolytic tank 12 via a pure water pipe 14, a SPE membrane, a sintered electrolytic anode and a sintered electrolytic cathode are provided in the electrolytic tank 12, the SPE membrane divides the tank body into a hydrogen chamber and an oxygen chamber, and the hydrogen chamber is in communication with the hydrogen water separator 13.

Specifically, the hydrogen water separator 13 is in communication with the pure water tank 9 through a pure water pipe 14, and is connected with the hydrogen storage tank 16 via a hydrogen pipe 15. As shown in FIG. 9, a hydrogen pressure sensor 161 is provided corresponding to the hydrogen tank 16, and the hydrogen pressure sensor 161 is connected with the control circuit board 8 for monitoring a hydrogen pressure parameter of the hydrogen tank 16.

Alternatively, a water tank cover 10 is further provided on the pure water tank 9, and a switch plate 7 is provided on the machine housing 1 corresponding to the water tank cover 10 for adding pure water into the pure water tank 9.

Preferably, as shown in FIG.9, the electrolytic hydrogen generation module further includes an oxygen-water separator 40, the oxygen chamber being in communication with the oxygen-water separator 40, the oxygen-water separator 40 delivering oxygen to the gas production filter 21 of the molecular sieve oxygen generation module via an oxygen pipe 19. The oxygen produced by the electrolytic hydrogen generation module is transported into the molecular sieve oxygen generation module, and the oxygen generation efficiency is improved.

Alternatively, as shown in FIGS. 7 and 8, one end of the hydrogen water separator 13 and the oxygen-water separator 40 outputs hydrogen and oxygen respectively through the hydrogen pipe 15 and the oxygen pipe 19, and the separated pure water is delivered back to the pure water tank 9 through a water return pipe 42.

Alternatively, as shown in FIG. 9, a water level/water temperature sensor 91 is provided corresponding to the pure water tank 9, the water level/water temperature sensor 91 is connected with the control circuit board 8 and is used for monitoring the working state of the pure water tank 9, and when the water level is low, a user is prompted to add water through an acousto-optic prompt, and if the water is not added in time, the machine will be turned off for protection; high water level indicates not to add water.

Alternatively, as shown in FIG. 9, the electrolytic hydrogen generation module further includes an ion exchange resin filter element 92, a circulation loop is formed between the pure water tank 9 and the ion exchange resin filter element 92, a water quality detection head 93 is provided on the circulation loop, and the water quality detection head 93 is connected with the control circuit board 8. The water quality detection head 93 is a TDS sensor, and when the TDS value is greater than 10 PPM, it is prompted to replace pure water, otherwise, the machine is turned off for giving an alarm.

Alternatively, as shown in FIG. 9, a voltage sensor 121 for monitoring the operating voltage of the single cells and a temperature sensor 122 for monitoring the temperature of the electrolytic tank 12 are provided corresponding to the electrolytic tank 12, and the voltage sensor 121 and the temperature sensor 122 are connected with the control circuit board 8. The operating temperature of the electrolytic tank 12 is generally 5-55°C, and if exceeding 60°C, the machine is turned off for giving an alarm; in addition, the electrolytic tank 12 operates at a voltage below 2.2 V, and the machine is turned off for giving an alarm when the voltage exceeds 3 V. As shown in FIG. 2, an electrolytic tank radiating fan 39 is provided corresponding to the electrolytic tank 12. Alternatively, as shown in FIG. 9, an orientation sensor 82 is provided in the machine housing 1 for monitoring an inclination angle of the machine housing, the orientation sensor 82 being connected with a control circuit board 8. The device needs to be placed horizontally for operation, and when the orientation sensor 82 detects that the device is tilted by more than 15 degrees, the machine is turned off for giving an alarm. The orientation sensor 82 may be a gyroscope, an electronic compass, an acceleration sensor and the like.

Alternatively, an oxygen check valve 29 is provided at an input of the oxygen storage tank 27 and a hydrogen check valve 32 is provided at an input end of the hydrogen storage tank 16.

[0001] Preferably, as shown in FIG. 9, the control circuit board 8 is provided to be connected with a wireless communication device 81, and the wireless communication device 81 is one or more of a Bluetooth communication module, a wireless RF communication module and a Wi-Fi communication module. Preferably, a Bluetooth 4.0 communication module is used for realizing data communication with a mobile terminal, and can be connected with a cloud server via the mobile terminal or directly connected with the cloud server.

Preferably, as shown in FIG. 9, a power-off alarm device 83 is integrally provided on the control circuit board 8, and when the device is powered off, an acousto-optic prompt is sent out to remind the user to take necessary measures so as to avoid injury to the user.

Embodiments of the present invention have been described in detail with reference to the accompanying drawings, but the present invention is not limited to the described embodiments. It will be apparent to a person skilled in the art that various changes, modifications, substitutions and variations can be made in the embodiments without departing from the spirit and scope of the present invention.

### Industrial applicability

The oxygen-hydrogen integrated machine of the present invention is provided with a molecular sieve oxygen generation module and an electrolytic hydrogen generation module, wherein pressure regulating valves are respectively provided at the output ends of oxygen and hydrogen so that the output pressures of oxygen and hydrogen are the same, and then oxygen and hydrogen are respectively mixed via a flow limiting hole; a volume ratio of oxygen to hydrogen is greater than 24:1, and the oxygen-hydrogen mixed gas is in an absolutely safe state by using the physical structure characteristics of the flow limiting hole; secondly, the volume ratio of the oxygen storage tank to the hydrogen storage tank is set to be greater than 24:1, and the ratio of the input oxygen and hydrogen is ensured to be in an absolute safe state before the oxygen and hydrogen are mixed; thirdly, the ratio of the oxygen output of the molecular sieve oxygen generation module and hydrogen output of the electrolytic hydrogen generation module is greater than 24:1, so that the ratio between the total oxygen generation amount and the hydrogen production amount of the device is in an absolute safe state, and the oxygen of the electrolytic hydrogen generation module is input to a gas production end of the molecular sieve oxygen generation module, improving the oxygen generation efficiency and further increasing the safety factor of the device, and avoiding a dangerous uncontrollable state of the existing oxygen generator and hydrogen generator combination or oxygen and hydrogen generator when oxygen and hydrogen are mixed by the above multiple safety restrictions.

## Claims

1. An oxygen-hydrogen integrated machine, comprising a machine housing, wherein a molecular sieve oxygen generation module and an electrolytic hydrogen generation module are provided in the machine housing, an oxygen-hydrogen interface is provided on one side of the machine housing, the molecular sieve oxygen generation module and the electrolytic hydrogen generation module respectively output oxygen and hydrogen at the same gas pressure by means of an oxygen pressure regulating valve and a hydrogen pressure regulating valve, an oxygen flow limiting hole and a hydrogen flow limiting hole are respectively provided at an output end of the oxygen pressure regulating valve and an output end of the hydrogen pressure regulating valve, and the oxygen and the hydrogen are in communication with the oxygen-hydrogen interface after passing through the oxygen flow limiting hole and the hydrogen flow limiting hole; and a ratio of the cross-sectional areas of the oxygen flow limiting hole and the hydrogen flow limiting hole is greater than 24:1.

2. The oxygen-hydrogen integrated machine according to claim 1, wherein the molecular sieve oxygen generation module and the electrolytic hydrogen generation module are respectively provided with an oxygen storage tank and a hydrogen storage tank, and the ratio of the volumes between the oxygen storage tank and the hydrogen storage tank is greater than 24: 1; an output end of the oxygen storage tank is provided with the oxygen pressure regulating valve, and an output end of the hydrogen storage tank is provided with the hydrogen pressure regulating valve.

3. The oxygen-hydrogen integrated machine according to claim 1 or 2, wherein a ratio of an oxygen output of the molecular sieve oxygen generation module to a hydrogen output of the electrolytic hydrogen generation module is greater than 24:1.

4. The oxygen-hydrogen integrated machine according to claim 1, wherein a gas-mixing valve is provided on the oxygen-hydrogen interface, input ends of the gas-mixing valve are respectively connected with oxygen and hydrogen, and an oxygen-hydrogen mixed gas is formed at the oxygen-hydrogen interface, and the volume concentration of hydrogen in the oxygen-hydrogen mixed gas is not greater than 4%.

5. The oxygen-hydrogen integrated machine according to claim 2, wherein an oxygen safety valve is provided between the oxygen storage tank and the oxygen pressure regulating valve, and a hydrogen safety valve is provided between the hydrogen storage tank and the hydrogen pressure regulating valve.

6. The oxygen-hydrogen integrated machine according to claim 4, wherein an output end of the gas-mixing valve is connected with a flow regulating valve for regulating an output of the oxygen-hydrogen mixed gas.

7. The oxygen-hydrogen integrated machine according to claim 6, wherein a rear end of the flow regulating valve is provided with a humidifier or an atomizer.

8. The oxygen-hydrogen integrated machine according to claim 1, wherein a control circuit board and a power supply device are provided in the machine housing, and the control circuit board is connected with a display screen, a power switch and a light-emitting diode (LED) indicator lamp provided on one side of the machine housing; the power supply device and the control circuit board are respectively connected with the molecular sieve oxygen generation module and the electrolytic hydrogen generation module.

9. The oxygen-hydrogen integrated machine according to claim 1, wherein the molecular sieve oxygen generation module comprises a compressor, a gas production filter connected with an input end of the compressor, and a plurality of molecular sieves connected with an output end of the compressor via a solenoid valve group, and the molecular sieves are connected with the oxygen storage tank.

10. The oxygen-hydrogen integrated machine according to claim 9, wherein the plurality of molecular sieves are provided in the machine housing via a mounting vertical plate, the compressor is provided in a mounting frame, and the solenoid valve group, a plurality of condensing fans and a condenser are provided on the mounting frame; the control circuit board and the power supply device are provided on the mounting frame via a circuit mounting board.

11. The oxygen-hydrogen integrated machine according to claim 1, wherein the electrolytic hydrogen generation module comprises an electrolytic tank, a pure water tank provided on an upper side of the electrolytic tank via a water tank mounting board, and a hydrogen-water separator, wherein the pure water tank is connected with the electrolytic tank via a pure water pipe, a solid polymer electrolyte (SPE) membrane, a sintered electrolytic anode and a sintered electrolytic cathode are provided in the electrolytic tank, the SPE membrane divides the tank body into a hydrogen chamber and an oxygen chamber, and the hydrogen chamber is in communication with the hydrogen-water separator.

12. The oxygen-hydrogen integrated machine according to claim 11, wherein the electrolytic hydrogen generation module further comprises an oxygen-water separator, the oxygen chamber being in communication with the oxygen-water separator, the oxygen-water separator delivering oxygen to a gas production filter of the molecular sieve oxygen generation module via an oxygen pipe.

13. The oxygen-hydrogen integrated machine according to claim 8, wherein an orientation sensor for monitoring an inclination angle of the machine housing is provided in the machine housing, the orientation sensor being connected with the control circuit board.

14. The oxygen-hydrogen integrated machine according to claim 2, wherein an oxygen check valve is provided at an input end of the oxygen storage tank, and a hydrogen check valve is provided at an input end of the hydrogen storage tank.

15. The oxygen-hydrogen integrated machine according to claim 8, wherein the control circuit board is provided to be connected with a wireless communication device, and the wireless communication device is one or more of a Bluetooth communication module, a wireless radio frequency (RF) communication module, and a Wi-Fi communication module.
